(19) Europäisches Patentamt
European Patent Office
Office européen des brevets

(11) EP 0 920 859 B1

(12) # FASCICULE DE BREVET EUROPEEN

(45) Date de publication et mention de la délivrance du brevet:
**11.04.2001 Bulletin 2001/15**

(51) Int Cl.$^7$: **A61K 7/42**

(21) Numéro de dépôt: **98402824.1**

(22) Date de dépôt: **13.11.1998**

(54) **Dispersion aqueuse comprenant un filtre UV du type organosiloxane à fonction benzalmalonate et un tensioactif cationique insoluble dans l'eau**

Wässrige Dispersion enthaltend als UV-Filter ein mit Benzalmalonat funktionalisiertes Organopolysiloxan und ein in Wasser unlösliches kationisches Tensid

Aqueous dispersion containing as UV-filter an organopolysiloxane functionalised with benzalmalonate and a water insoluble cationic tensid

(84) Etats contractants désignés:
**CH DE ES FR GB IT LI**

(30) Priorité: **03.12.1997 FR 9715243**

(43) Date de publication de la demande:
**09.06.1999 Bulletin 1999/23**

(73) Titulaire: **L'OREAL**
**75008 Paris (FR)**

(72) Inventeurs:
- **Dubief, Claude**
  **77150 Le Chesnay (FR)**
- **Cauwet-Martin, Danièle**
  **75011 Paris (FR)**

(74) Mandataire: **Miszputen, Laurent**
**L'OREAL-DPI**
**6 rue Bertrand Sincholle**
**92585 Clichy Cédex (FR)**

(56) Documents cités:
**EP-A- 0 389 337** **EP-A- 0 583 888**
**EP-A- 0 709 080**

Remarques:
Le dossier contient des informations techniques présentées postérieurement au dépôt de la demande et ne figurant pas dans le présent fascicule.



Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Printed by Jouve, 75001 PARIS (FR)

## Description

L'invention concerne une composition sous forme de dispersion aqueuse, caractérisée en ce qu'elle comprend dans un milieu cosmétiquement acceptable au moins un agent filtrant le rayonnement ultraviolet, liposoluble du type organosiloxane à fonction benzalmalonate et au moins un tensioactif cationique insoluble dans l'eau ainsi que ses utilisations en cosmétique notamment pour la protection des fibres kératiniques et de leur couleur naturelle ou teinte artificiellement, en particulier des cheveux contre les effets néfastes des rayons UV.

On sait depuis longtemps que la lumière en particulier les ultraviolets dégradent les propriétés cosmétiques et/ou mécaniques des cheveux. Les cheveux sont alors ternes, rêches et cassants. Les cheveux, contrairement à la peau, éclaircissent.

On recherche depuis de nombreuses années, dans l'industrie cosmétique des substances permettant de protéger les cheveux des dégradations engendrées par les agressions atmosphériques telles que les rayonnements UV en particulier les rayonnements solaires. D'autre part, on cherche à obtenir en même temps de bonnes propriétés cosmétiques relatives aux cheveux comme la douceur au toucher, un bon démêlage ou la brillance.

Dans cette optique, on a déjà proposé d'utiliser les filtres solaires utilisés pour la photoprotection de la peau dans les formulations pour le traitement et le soin capillaire. Cependant, la structure de la peau et des cheveux sont très différentes et la demanderesse a ainsi constaté que la plupart des filtres utilisés dans les compositions pour la peau n'étaient pas efficaces pour la protection des cheveux.

On distingue parmi les filtres solaires habituellement utilisés en cosmétique, les filtres hydrosolubles généralement porteurs de groupements ioniques : anioniques ou cationiques et les filtres liposolubles ayant en général un caractère non-ionique.

Les filtres hydrosolubles du type anionique, sont généralement difficiles à mettre en oeuvre dans les formulations capillaires contenant de nombreux types d'agents de conditionnement comme des polymères cationiques ou des tensioactifs cationiques ou bien des épaississants avec lesquels ils sont incompatibles.

Les compositions capillaires contenant des filtres hydrosolubles du type cationique en présence d'agents de conditionnement de même polarité ont une activité protectrice des cheveux contre les effets des rayons UV très limitée en raison d'une compétitivité entre les différentes entités cationiques pour la fixation sur les sites anioniques des fibres kératiniques. D'autre part, les filtres hydrosolubles ne sont généralement pas appropriés pour les compositions conditionnantes dites rincées ; ils s'éliminent facilement des cheveux lors de l'étape finale de rinçage.

Les filtres liposolubles qui sont généralement utilisés dans les compositions aqueuses de traitement capillaire de l'art antérieur nécessitent une solubilisation dans une phase grasse et/ou une stabilisation à l'aide d'agents émulsionnants spécifiques.

On connaît notamment des formulations capillaires rincées renfermant des filtres UV liposolubles, constituées de trois phases : une première phase aqueuse, une deuxième phase lamellaire à base de tensioactif cationique et d'alcool gras à longue chaîne et une troisième phase grasse contenant les filtres UV liposolubles. Ces compositions conduisent généralement, après application et rinçage à l'eau, à une mauvaise répartition du filtre liposoluble sur la chevelure et à une quantité de filtre déposée insuffisante pour assurer une bonne protection vis-à-vis des rayonnements UV.

Pour pallier à ces inconvénients, on a proposé, dans la demande de brevet EP-A0419164, des compositions capillaires de conditionnement comprenant une phase aqueuse et une phase lamellaire contenant au moins un tensioactif cationique conditionneur et un filtre liposoluble sous forme d'huile tel que le 2-éthylhexyl-p-méthoxy cinnamate (vendu sous le nom commercial PARSOL MCX), le 2-éthylhexyl-para-diméthylaminobenzoate (vendu sous le nom commercial ESCALOL 507) et/ou le dihydroxypropyl-p-aminobenzoate (vendu sous le nom commercial AMERSCREEN P).

On connaît également dans la demande EP-A-389 337 des polyorganosiloxanes à fonction hydroxybenzophénone lipososubles comme filtres UV dans diverses formulations cosmétiques, notamment capillaires. Ces polymères filtres sont en particulier utilisés pour préserver les propriétés mécaniques des cheveux et la dégradation de leur couleur. In exemple de mousse capillaire contient l'un de ces polyorganosiloxanes en présence d'un tensio-actif cationique: le chlorure de diméthyl dialkyl (suif) ammonium. On connaît également dans la demande EP-A-583 888 des copolymères hydrocarbonés d'au moins un monomère filtrant les radiations UV-A, un monomère hydrophile et d'au moins un monomère filtrant les radiations UV-B et leurs utilisations dans des formulations cosmétiques détergentes tels que des shampooings pouvant contenir des tensio-actifs cationiques.

La demanderesse a trouvé de façon surprenante et inattendue que l'utilisation d'un tensioactif cationique insoluble dans l'eau dans une dispersion aqueuse comprenant un agent filtrant le rayonnement ultraviolet liposoluble du type organosiloxane à fonction benzalmalonate permettait d'obtenir directement des dispersions aqueuses desdits filtres liposolubles, stables et homogènes ne présentant pas tous les inconvénients techniques énumérés précédemment. La Demanderesse a découvert que les formulations ainsi obtenues pouvaient être utilisées dans toutes les formes de formulations capillaires et permettaient d'obtenir des quantités de filtre déposées sur les cheveux suffisantes

pour obtenir une bonne protection de la chevelure contre les agressions des rayons UV et de la couleur des cheveux naturelle ou teinte artificiellement. De plus, elles apportent d'excellentes propriétés cosmétiques notamment au niveau du démêlage, de la douceur ou de la brillance. La Demanderesse a découvert que les dispersions aqueuses de l'invention pouvaient être utilisées plus particulièrement dans les compositions capillaires dont l'application sur les cheveux était suivie d'un rinçage à l'eau et qu'elles permettaient d'obtenir à la fois de meilleures performances cosmétiques, une plus grande quantité de filtre déposée sur les cheveux, en particulier sur cheveux peu sensibilisés ainsi qu'une meilleure répartition et une plus grande rémanence au rinçage par rapport à celles obtenues avec les compositions de l'art antérieur contenant un filtre liposoluble classique.

La présente invention a pour objet une composition cosmétique ou dermatologique sous forme de dispersion aqueuse comprenant dans un milieu cosmétiquement acceptable au moins un tensioactif cationique insoluble dans l'eau et au moins un filtre UV liposoluble du type organosiloxane à fonction benzalmalonate.

La présente invention a pour objet l'utilisation de tensioactifs cationiques insolubles dans l'eau dans, ou pour la fabrication d'une composition capillaire comprenant au moins au moins un filtre UV liposoluble du type organosiloxane à fonction benzalmalonate, destinée à protéger les cheveux contre les effets des rayonnements UV et la couleur des cheveux naturelle ou teinte artificiellement.

La présente invention a pour objet l'utilisation de tensioactifs cationiques insolubles dans l'eau dans, ou pour la fabrication d'une composition capillaire comprenant au moins au moins un filtre UV liposoluble du type organosiloxane à fonction benzalmalonate pour améliorer le dépôt et/ou la fixation dudit filtre UV sur les cheveux.

Par utilisation capillaire, on entend selon la présente invention l'application de la composition sur les cheveux pour leur lavage et /ou leur traitement et/ou leur conditionnement.

Par tensioactif insoluble dans l'eau, on entend selon la présente invention, les tensioactifs qui à la concentration de 1 % M. A. à 25 °C ne conduisent pas à une solution transparente isotrope.

Selon la présente invention, les agents filtrant les radiations UV liposolubles du type organosiloxane à fonction benzalmalonate sont choisis de préférence parmi ceux comportant au moins des unités répondant à l'une des deux formules suivantes (1) et (2) :

$$O_{\frac{3-c}{2}}\,Si(R'')_c - M - O - C_6R'''_2H_2 - CH{=}C - \left[C(O)OR''''\right]_2 \qquad (1)$$

$$O_{\frac{3-c}{2}}\,Si(R'')_c - M' - O - C_6R'''_2H_2 - CH{=}C - \left[C(O)OR''''\right]_2 \qquad (2)$$

dans lesquelles :

- M désigne un groupe de structure:

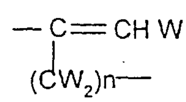

ou

$$-CW{=}CH-(CW_2)_n-$$

- M' désigne un groupe de structure :

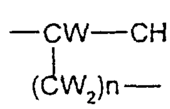

ou

$$-CW_2-CHW-(CW_2)n-$$

les autres unités de la silicone présentes étant de structure:

$$Qd - Si - O_{\frac{4-d}{2}} \qquad (3)$$

où R" désigne un radical alkyle en $C_1$-$C_8$ ou aryle ; W est un hydrogène ou un radical alkyle en $C_1$-$C_5$ ; R"' est un hydrogène ou un radical alkyle en $C_1$-$C_5$ ou un radical OW ; R"" désigne un groupe alkyle en $C_1$-$C_5$ ; Q désigne un hydrogène, un radical hydrocarboné monovalent en $C_1$-$C_8$ ou un groupe hydrocarboné halogéné ; c vaut 0, 1 ou 2 ; d vaut 0, 1, 2 ou 3 et n a une valeur de 1 à 6 ; sous réserve que le groupe -M-O- ou -M'-O- soit relié au noyau aromatique en position méta ou para par rapport au groupe :

$$-CH=C-\left[C(O)OR''''\right]_2$$

et que les deux groupes R"' occupent les autres positions restantes du noyau aromatique.

Ces silicones filtres sont décrites ainsi que leurs procédés de préparation dans les demandes de brevet EP-A-0 392 882, EP-0 538 431, EP-A-0 709 080 et WO92/20690.

Dans une forme particulièrement préférée de réalisation de l'invention, une famille de composés particulièrement recherchée est celle définie par les silicones benzalmalonates choisies dans le groupe constitué par :

(i) les silicones répondant à la formule (4) suivante :

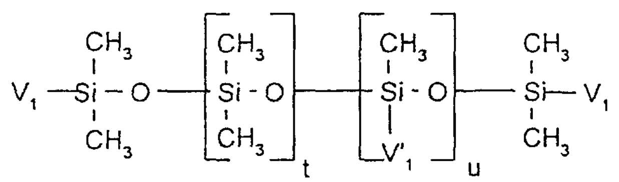

(4)

dans laquelle :

$V'_1$ désigne le groupe de structure :

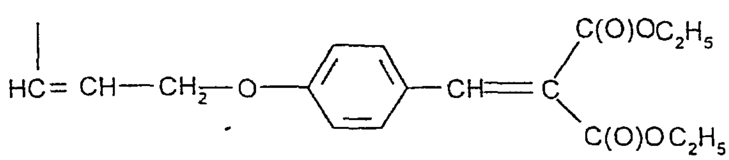

$V_1$ désigne $CH_3$ ou $V'_1$ ;

(ii) les silicones répondant à la formule (5) suivante :

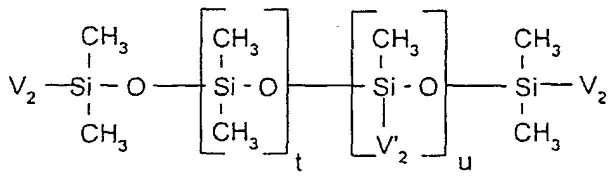

(5)

V'$_2$ désigne le groupe de structure:

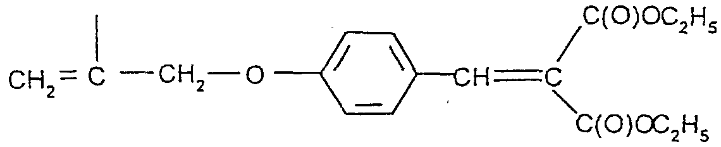

V$_2$ désigne $CH_3$ ou V'$_2$ ;

(iii) leurs mélanges ;

avec $0 \leq t \leq 100$ et $0 \leq u \leq 20$ sous réserve que :

- lorsque $V_1 = V'_1$ et/ou $V_2 = V'_2$ alors $u = 0$ ; et
- lorsque $V_1 = CH_3$ et/ou $V_2 = CH_3$ alors $1 \leq u \leq 20$.

Les organosiloxanes filtres de l'invention sont utilisés de préférence dans des quantités au moins égales à 0,05% en poids et généralement allant de 0,05 à 10% en poids et plus particulièrement de 0,1 à 6% en poids par rapport au poids de la composition.

Les agents tensioactifs cationiques insolubles dans l'eau sont, selon l'invention, de préférence choisis dans le groupe constitué par les sels d'ammonium quaternaire , les amines grasses et leurs sels.

Parmi les amines grasses utilisables selon l'invention, on peut citer la dioctylamine, la stéaryl-diméthylamine, la palmityldiméthylamine, l'oléocétyldiméthylamine et les amidoamines telles que la stéarylamidoéthyldiéthylamine, la béhénylamidopropyldiméthylamine, la stéaryl-amidopropyldiméthylamine, l'oléylamidopropyldiméthylamine, la stéarylamidoéthyldiméthylamine.

Et plus particulièrement, les agents tensioactifs cationiques de l'invention sont choisis parmi :

- les sels d'ammonium quaternaire répondant à la formule générale (I) suivante:

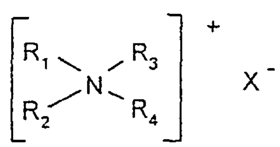

(I)

dans laquelle les radicaux $R_1$ à $R_4$, qui peuvent être identiques ou différents, représentent un radical aliphatique substitué ou non comportant de 1 à environ 30 atomes de carbone tels que alkyle, alcoxy, polyoxyalkylène($C_2$-$C_6$), alkylamide, alkyl($C_{12}$-$C_{22}$)amidoalkyle($C_2$-$C_6$), alkyl($C_{12}$-$C_{22}$)acétate, hydroxyalkyle ou un radical aromatique tel que aryle ou alkylaryle ; X est un anion choisi dans le groupe des halogénures, phosphates, acétates, lactates, alkylsulfates, alkyl-ou-alkylarylsulfonates ;

- les sels d'ammonium quaternaire de l'imidazolinium, comme par exemple celui de formule (II) suivante :

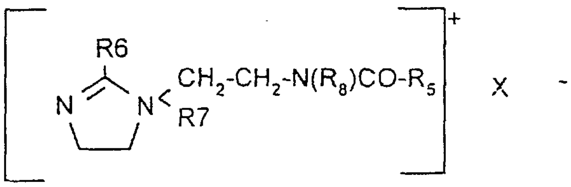 (II)

dans laquelle $R_5$ représente un radical alcényle ou un alkyle comportant de 8 à 30 atomes de carbone tels que des dérivés des acides gras du suif ; $R_6$ représente un atome d'hydrogène, un radical alkyle en $C_1$-$C_4$ ou un radical alcényle ou un alkyle comportant de 8 à 30 atomes de carbone; $R_7$ représente un radical alkyle en $C_1$-$C_4$ ; $R_8$ représente un atome d'hydrogène ou un radical alkyle en $C_1$-$C_4$ ; X est un anion choisi dans le groupe des halogénures, phosphates, acétates, lactates, alkylsulfates, alkyl-ou-alkylarylsulfonates ; $R_5$ et $R_6$ représentent de préférence un mélange de radicaux alcényle et/ou alkyle comportant de 12 à 21 atomes de carbone par exemple dérivés des acides gras du suif, tel que le produit vendu sous la marque «REWOQUAT W 75PG» par la société REWO.

- les sels de diammonium quaternaire de formule (III) :

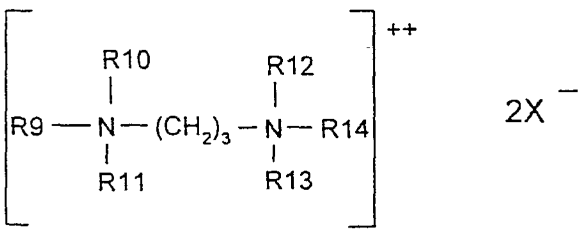 (III)

dans laquelle $R_9$ désigne un radical aliphatique comportant environ de 16 à 30 atomes de carbone, $R_{10}$, $R_{11}$, $R_{12}$, $R_{13}$, et $R_{14}$ sont choisis parmi l'hydrogène ou un radical alkyle comportant de 1 à 4 atomes de carbone, et X est un anion choisi dans le groupe des halogénures, acétates, phosphates, sulfates, nitrates et méthylsiulfates. De tels sels de diammonium quaternaire comprennent notamment le dichlorure de propanesuif diammonium.

Parmi les sels d'ammonium quaternaire de formule (I) on utilisera plus particulièrement, d'une part, les chlorures de tétraalkylammonium comme par exemple les chlorures de dialkyldiméthylammonium ou d'alkyltriméthylammonium, dans lesquels le radical alkyl comporte environ de 12 à environ 30 atomes de carbone, en particulier les chlorures de distéaryldiméthylammonium ou de benzyldiméthylstéarylammonium.

Les tensioactifs cationiques insolubles dans l'eau sont utilisés de préférence dans des quantités généralement allant de 0,05 à 10% en poids et plus particulièrement de 0,1 à 6% en poids par rapport au poids de la composition.

Selon'une forme particulièrement préférée de l'invention, les compositions de l'invention ont un pH supérieur ou égal à 3, plus préférentiellement un pH allant de 4 à 8 et encore plus préférentiellement de 4 à 6.

Les compositions de l'invention comportent généralement au moins une phase aqueuse et une phase grasse constituée, soit uniquement du ou des organosiloxanes filtres de l'invention, soit d'un mélange de celui-ci (ou de ceux-ci) avec un ou plusieurs corps gras choisis parmi les huiles minérales, végétales, animales ou synthétiques ; les huiles de silicone à structure linéaire ou cyclique comme les polyalkyl-siloxanes, les polyarylsiloxanes, les polyalkylarylsiloxanes ou les polyorganosiloxanes modifiés par des groupements organofonctionnels non-chromophores ; les cires, les résines ou les gommes de silicone ainsi que leurs mélanges.

Les compositions concernées par l'invention peuvent être préparées selon les techniques bien connues de l'homme de l'art, en particulier celles destinées à la préparation de dispersions à phase continue aqueuse telles que les émulsions de type huile-dans-eau, les gels et les gels-crèmes. Elles peuvent également se présenter sous forme d'émulsion, simple ou complexe (H/E, E/H, H/E/H ou E/H/E).

Les compositions selon l'invention peuvent se présenter sous forme de crèmes, de laits, de gels, de gels-crèmes, de dispersions vésiculaires non ioniques, de lotions. Les compositions conformes à l'invention peuvent également se présenter sous forme de spray ou être pressurisées dans des dispositifs aérosols.

La phase aqueuse peut comporter en plus de l'eau un ou plusieurs solvants organiques cosmétiques classiques tels qu'un alcool inférieur comportant 1 à 4 atomes de carbone et de préférence l'éthanol ou l'isopropanol ou bien d'autres alcools tels que les alkylèneglycols ou les éthers de glycols.

Les compositions conformes à l'inventions peuvent également contenir tout autre additif habituellement appliqué sur les fibres kératiniques et en particulier les cheveux humains tels que par exemple des colorants, des tensioactifs, des polymères, des épaississants, des agents de conditionnement, des opacifiants, des nacrants, des antipelliculaires, des antichutes, des céramides, des vitamines, des anti-oxydants, des parfums, des conservateurs, des adoucissants ainsi que d'autres agents filtrants.

Bien entendu, l'homme de l'art veillera à choisir le ou les éventuels composés complémentaires cités ci-dessus et/ou leurs quantités de manière telle que les propriétés avantageuses attachées intrinsèquement à l'association [organosiloxane filtre + tensioactif cationique] conforme à l'invention ne soient pas, ou substantiellement pas, altérées par la ou les adjonctions envisagées.

Les compositions de l'invention destinées à être appliquées sur des fibres kératiniques et en particulier des cheveux humains, sont des produits rincés tels que des shampooings, des après-shampooings, à appliquer avant ou après coloration ou décoloration, avant ou après permanente ou défrisage ou bien des produits non-rincés tels qu'une lotion ou un gel coiffant ou traitant, une lotion ou un gel pour le brushing ou la mise en plis ; des produits de permanente ou de défrisage ; des produits de coloration ou décoloration des cheveux.

La présente invention a également pour objet un procédé de traitement cosmétique pour la protection des cheveux contre les effets des rayons UV et de la couleur naturelle ou teinte artificiellement des cheveux, consistant à appliquer sur les cheveux une quantité efficace d'une composition sous forme de dispersion aqueuse comprenant au moins au moins un filtre UV liposoluble du type organosiloxane à fonction benzalmalonate et au moins un tensioactif cationique insoluble dans l'eau tel que défini ci-dessus.

Les exemples qui suivent servent à illustrer l'invention sans toutefois présenter un caractère limitatif.

**EXEMPLE 1**: **Formulation solaire**

- Organosiloxane à fonction benzalmalonate de formule (4) ou (5) 1g
- Chlorure de béhényl diméthyl benzyl ammonium vendu sous le nom AMMONYX 4022 (GOLDSCHMIDT) 5g MA
- Conservateur, Parfum qs
- Eau qsp 100g

**EXEMPLE 2** : **Formulation solaire**

- Organosiloxane à fonction benzalmalonate de formule (4) ou (5) 0,5g
- Méthosulfate de 1-méthyl 2-suif 3-suifamidoéthyl imidazolium à 75 % dans le propylène glycol vendu sous le nom REWOQUAT W75PG (REWO) 2g MA
- Conservateur, Parfum qs
- Eau qsp 100g

**EXEMPLE COMPARATIF 2**

- 2-éthylhexyl-p-méthoxy cinnamate sous forme d'huile (vendu sous le nom commercial PARSOL MCX) 0,5g
- Méthosulfate de 1-méthyl 2-suif 3-suifamidoéthyl imidazolium à 75% dans le propylène glycol vendu sous le nom REWOQUAT W75PG (REWO) 2g MA
- Conservateur, Parfum qs
- Eau qsp 100g

**ESSAIS COMPARATIFS** :

**1. Dépôt de filtre sur cheveux**

On a mesuré le dépôt de filtre siliconé de l'invention sur des cheveux naturels après application de la composition de l'exemple 2 et rinçage à l'eau. On a comparé ce dépôt à celui du 2-éthylhexyl-p-méthoxy cinnamate sous forme d'huile obtenu avec la composition de l'exemple comparatif 2.

**PROTOCOLE** :

On mesure la quantité de filtre UV déposé sur les cheveux en mesurant l'absorbance du filtre UV à la longueur d'onde caractéristique au spectrophotomètre. On calcule la concentration du filtre à partir d'une courbe étalon préétablie.

Les mèches de cheveux utilisées sont constituées de cheveux naturels châtains, 90% blancs, de poids d'environ 1g. Le spectrophotomètre utilisé est du type SHIMATZU UV 2100 (ROUCAIRE). Le filtre étudié fixé sur les cheveux est extrait dans du dichlorométhane. Les tests sont effectués sur 4 échantillons pour chaque formule étudiée et 2 échantillons pour la formule placébo témoin.

Chaque mèche de 1 g est introduite dans un pilulier de 30 ml. On ajoute 25 ml de dichlorométhane. On place les piluliers sous agitation mécanique pendant 2 heures ; 170 déplacements/minute (agitateur HEIDOLF PROMAX 2020). On remonte la mèche avec une pince au-dessus du niveau liquide ; on la laisse égoutter 15 secondes.

On établit une courbe étalon avec des quantités de 1 à 10 mg de filtre ; la réponse doit être linéaire du type Y = aX +b avec X représentant la concentration du filtre dans la solution extraite en mg/g de cheveux. On pèse les quantités de filtres dans des fioles jaugées de 100 ml que l'on complète avec le solvant. Après solubilisation, on mesure dans des cuves 115B-QS (trajet optique 10mm) pour spectrophotomètre l'absorbance du filtre dans chaque solution à sa longueur d'onde caractéristique ainsi que celle d'une solution témoin à base du solvant. On effectue ensuite pour chaque échantillon un dosage du filtre déposé sur la mèche avec la solution d'extraction de la mèche traitée par la formulation solaire contenant le filtre. On calcule l'absorbance moyenne du filtre dans 25ml de dichlorométhane (Densité optique). On déduit à partir de la courbe étalon la concentration du filtre déposé sur les cheveux).

Les résultats sont résumés dans le tableau suivant :

| COMPOSITION | Courbe étalon obtenue | Densité optique moyenne | Quantité de filtre déposé (mg/g de cheveux) |
|---|---|---|---|
| **Exemple 2** | Y = 0,02 + 0,77X | 1,68 | 2,14 |
| **Exemple comparatif 2** | Y = 0,6 + 3,84X | 2,35 | 0,6 |

Le composé filtre selon l'invention se dépose plus de 3 fois plus que le filtre liposoluble classique 2-éthylhexyl-p-méthoxy cinnamate.

2. **Propriétés cosmétiques**

10 personnes témoins selon un test d'évaluation sensorielle ont jugé que la composition de l'exemple 2 selon l'invention après application sur cheveux conduisait à un toucher nettement moins gras, moins chargé et plus agréable par rapport à la composition de l'exemple comparatif 2.

## Revendications

1. Composition cosmétique ou dermatologique sous forme de dispersion aqueuse, caractérisée par le fait qu'elle comprend dans un milieu cosmétiquement acceptable au moins un tensioactif cationique insoluble dans l'eau et au moins un filtre UV liposoluble du type organosiloxane à fonction benzalmalonate.

2. Composition selon la revendication 1, selon laquelle l'agent filtrant les radiations UV liposoluble du type organosiloxane à fonction benzalmalonate comporte au moins des unités répondant à l'une des deux formules suivantes (1) et (2) :

$$O_{\frac{3-c}{2}}Si(R'')_c - M - O - C_6R'''_2H_2 - CH{=}C - [C(O)OR'''']_2 \qquad (1)$$

$$O_{\frac{3-c}{2}}Si(R'')c—M'—O—C_6R'''_2H_2—CH{=}C—[C(O)OR'''']_2 \quad (2)$$

dans lesquelles :

- M désigne un groupe de structure :

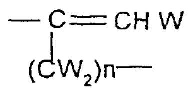

ou

$$—CW{=}CH—(CW_2)n—$$

- M' désigne un groupe de structure :

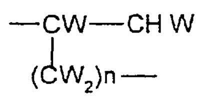

ou

$$—CW_2—CHW—(CW_2)n—$$

les autres unités de la silicone présentes étant de structure :

$$Qd—Si—O_{\frac{4-d}{2}} \quad (3)$$

où R" désigne un radical alkyle en $C_1$-$C_8$ ou aryle ; W est un hydrogène ou un radical alkyle en $C_1$-$C_5$ ; R''' est un hydrogène ou un radical alkyle en $C_1$-$C_5$ ou un radical OW ; R'''' désigne un groupe alkyle en $C_1$-$C_5$ ; Q désigne un hydrogène, un radical hydrocarboné monovalent en $C_1$-$C_8$ ou un groupe hydrocarboné halogéné ; c vaut 0, 1 ou 2 ; d vaut 0, 1, 2 ou 3 et n a une valeur de 1 à 6 ; sous réserve que le groupe -M-O- ou -M'-O- soit relié au noyau aromatique en position méta ou para par rapport au groupe:

$$—CH{=}C—[C(O)OR'''']_2$$

et que les deux groupes R''' occupent les autres positions restantes du noyau aromatique.

**3.** Composition selon la revendication 1, selon laquelle l'agent filtrant les radiations UV liposoluble du type organosiloxane à fonction benzalmalonate est choisi dans le groupe constitué par :

(i) les silicones répondant à la formule (4) suivante:

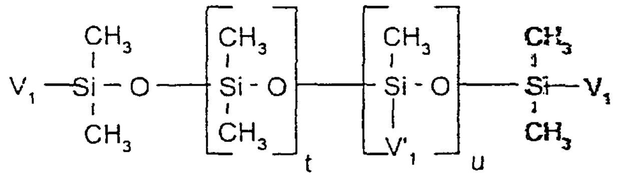

(4)

dans laquelle:

$V'_1$ désigne le groupe de structure:

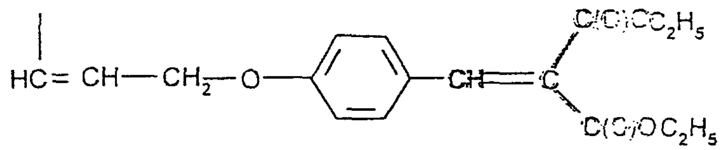

$V_1$ désigne $CH_3$ ou $V'_1$ ;

(ii) les silicones répondant à la formule (5) suivante:

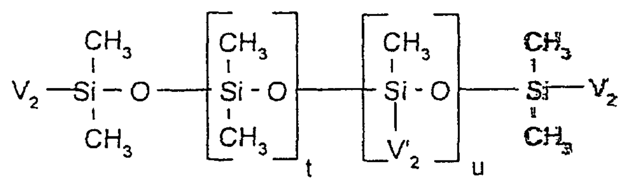

(5)

$V'_2$ désigne le groupe de structure:

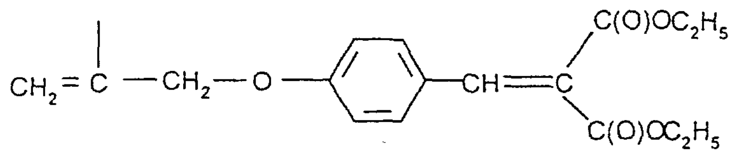

$V_2$ désigne $CH_3$ ou $V'_2$;

(iii) leurs mélanges ;

avec $0 \leq t \leq 100$ et $0 \leq u \leq 20$ sous réserve que :

- lorsque $V_1 = V'_1$ et/ou $V_2 = V'_2$ alors $u = 0$ ; et
- lorsque $V_1 = CH_3$ et/ou $V_2 = CH_3$ alors $1 \leq u \leq 20$.

**4.** Composition selon l'une quelconque des revendications 1 à 3, selon laquelle l'agent filtrant les radiations UV liposoluble du type organosiloxane à fonction benzalmalonate est présent allant de 0,05 à 10% en poids et plus particulièrement de 0,1 à 6% en poids par rapport au poids de la composition.

**5.** Composition selon l'une quelconque des revendications 1 à 4, selon laquelle les agents tensioactifs cationiques insolubles dans l'eau sont choisis dans le groupe constitué par les sels d'ammonium quaternaire , les amines grasses et leurs sels.

**6.** Composition selon l'une quelconque des revendications 1 à 5, selon laquelle les amines grasses sont choisis parmi la dioctylamine, la stéaryldiméthylamine, la palmityldiméthylamine, l'oléocétyldiméthylamine et les amidoamines telles que la stéarylamidoéthyldiéthylamine, la béhénylamidopropyldiméthylamine, la stéarylamidopropyldiméthylamine, l'oléylamidopropyldiméthylamine, la stéarylamidoéthyldiméthylamine.

**7.** Composition selon la revendication 5, selon laquelle les agents tensioactifs cationiques insolubles dans l'eau sont choisis parmi:

- les sels d'ammonium quaternaire répondant à la formule générale (I) suivante:

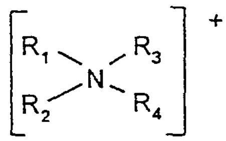

$X^-$ (I)

dans laquelle les radicaux $R_1$ à $R_4$, qui peuvent être identiques ou différents, représentent un radical aliphatique substitué ou non comportant de 1 à environ 30 atomes de carbone tels que alkyle, alcoxy, polyoxyalkylène ($C_2$-$C_6$), alkylamide, alkyl($C_{12}$-$C_{22}$)amidoalkyle($C_2$-$C_6$), alkyl($C_{12}$-$C_{22}$)acétate, hydroxyalkyle ou un radical aromatique tel que aryle ou alkylaryle; X est un anion choisi dans le groupe des halogénures, phosphates, acétates, lactates, alkylsulfates, alkyl-ou-alkylarylsulfonates ;

- les sels d'ammonium quaternaire de l'imidazolinium, comme par exemple celui de formule (II) suivante:

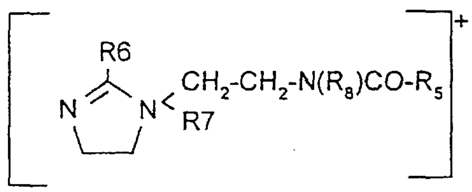

$X^-$ (II)

dans laquelle $R_5$ représente un radical alcényle ou un alkyle comportant de 8 à 30 atomes de carbone tels que des dérivés des acides gras du suif; $R_6$ représente un atome d'hydrogène, un radical alkyle en $C_1$-$C_4$ ou un radical alcényle ou un alkyle comportant de 8 à 30 atomes de carbone; $R_7$ représente un radical alkyle en $C_1$-$C_4$ ; $R_8$ représente un atome d'hydrogène ou un radical alkyle en $C_1$-$C_4$ ; X est un anion choisi dans le groupe des halogénures, phosphates, acétates, lactates, alkylsulfates, alkyl-ou-alkylarylsulfonates ;

- les sels de diammonium quaternaire de formule (III):

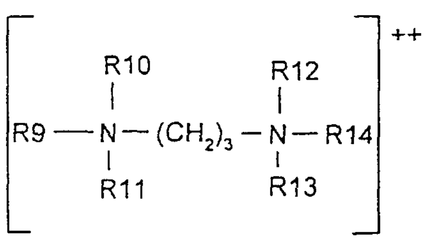

$2X^-$ (III)

dans laquelle $R_9$ désigne un radical aliphatique comportant environ de 16 à 30 atomes de carbone, $R_{10}$, $R_{11}$, $R_{12}$, $R_{13}$, et $R_{14}$ sont choisis parmi l'hydrogène ou un radical alkyle comportant de 1 à 4 atomes de carbone, et X est un anion choisi dans le groupe des halogénures, acétates, phosphates, sulfates, nitrates et méthylsulfates.

**8.** Composition selon l'une quelconque des revendications 1 à 7, selon laquelle les tensioactifs cationiques insolubles dans l'eau sont utilisés dans des quantités allant de 0,05 à 10% en poids et plus particulièrement de 0,1 à 6% en

poids par rapport au poids de la composition.

**9.** Composition selon l'une quelconque des revendications 1 à 8, selon laquelle le pH varie de 3 à 8 et plus préférentiellement de 4 à 6.

**10.** Composition selon l'une quelconque des revendications 1 à 9, caractérisée par le fait qu'elle comporte au moins une phase aqueuse et une phase grasse constituée, soit uniquement de l'organosiloxane filtre, soit par un mélange de celui-ci avec un ou plusieurs corps gras choisis parmi les huiles minérales, végétales, animales ou synthétiques ; les huiles de silicone à structure linéaire ou cyclique ou bien les cires, les résines ou les gommes de silicone ainsi que leurs mélanges.

**11.** Composition selon l'une quelconque des revendications 1 à 10, caractérisée par le fait qu'elle se présente sous forme de crème, de lait, de gel, de gel-crème, de dispersion vésiculaire non ionique, de lotion, de spray ou qu'elle est pressurisée dans un dispositif aérosol.

**12.** Composition selon l'une quelconque des revendications 1 à 11, caractérisée par le fait qu'elle contient en plus un ou plusieurs additifs cosmétiques choisis parmi des colorants, des tensioactifs, des polymères, des épaississants, des agents de conditionnement, des opacifiants, des nacrants, des anti-pelliculaires, des antichutes, des céramides, des vitamines, des anti-oxydants, des parfums, des conservateurs, des adoucissants ainsi que d'autres agents filtrants.

**13.** Composition selon l'une quelconque des revendications 1 à 12, caractérisée par le fait qu'elle est utilisée comme produit capillaire.

**14.** Procédé de traitement cosmétique pour la protection des cheveux contre les effets des rayons UV et de leur couleur naturelle ou teinte artificiellement, consistant à appliquer sur les cheveux une quantité efficace d'une composition telle que définie selon l'une quelconque des revendications 1 à 13.

**15.** Utilisation d'un tensioactif cationique insoluble dans l'eau tel que défini dans l'une quelconque des revendications 1 à 7, dans ou pour la fabrication d'une composition capillaire sous forme de dispersion aqueuse comprenant au moins au moins un filtre UV liposoluble du type organosiloxane à fonction benzalmalonate tel que définie dans l'une quelconque des revendications 1 à 3, dans le but de protéger les cheveux et leur couleur naturelle ou teinte artificiellement contre les effets des rayonnements UV.

**16.** Utilisation d'un tensioactif cationique insoluble dans l'eau tel que défini dans l'une quelconque des revendications 1 à 7, dans ou pour la fabrication d'une composition capillaire sous forme de dispersion aqueuse comprenant au moins un filtre UV liposoluble du type organosiloxane à fonction benzalmalonate tel que définie dans l'une quelconque des revendications 1 à 3, pour améliorer le dépôt et/ou la fixation dudit filtre UV sur les cheveux.

**Patentansprüche**

**1.** Kosmetische oder dermatologische Zusammensetzung, die in Form einer wäßrigen Dispersion vorliegt, dadurch gekennzeichnet, daß sie in einem kosmetisch akzeptablen Medium mindestens einen in Wasser unlöslichen, kationischen grenzflächenaktiven Stoff und mindestens ein fettlösliches UV-Filter vom Typ Organosiloxan mit Benzalmalonatgruppe enthält.

**2.** Zusammensetzung nach Anspruch 1, wobei das fettlösliche UV-Filter vom Typ Organosiloxan mit Benzalmalonatgruppe zumindest Einheiten aufweist, die einer der beiden folgenden Formeln (1) und (2) entsprechen:

$$O_{\frac{3-c}{2}}Si(R'')_c - M - O - C_6R'''_2H_2 - CH{=}C - \left[C(O)OR''''\right]_2 \qquad (1)$$

$$O_{\frac{3-c}{2}}Si(R'')_c — M' — O — C_6R'''_2H_2 — CH{=\!=}C — \left[C(O)OR''''\right]_2 \qquad (2),$$

worin bedeuten:

- M eine Gruppe der Formel:

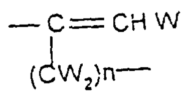

oder

$$—CW{=}CH—(CW_2)_n—$$

- M' eine Gruppe der Formel:

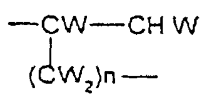

oder

$$—CW_2—CHW—(CW_2)_n—$$

wobei die anderen Einheiten des Silicons die folgende Struktur aufweisen:

$$Qd — Si — O_{\frac{4-d}{2}} \qquad (3)$$

worin bedeuten: R'' eine $C_{1-8}$-Alkylgruppe oder Aryl; W Wasserstoff oder eine $C_{1-5}$-Alkylgruppe; R''' Wasserstoff, eine $C_{1-5}$-Alkylgruppe oder eine Gruppe OW; R'''' eine $C_{1-5}$-Alkylgruppe; Q Wasserstoff, eine einwertige Kohlenwasserstoffgruppe mit 1 bis 8 Kohlenstoffatomen oder eine halogenierte Kohlenwasserstoffgruppe; c 0, 1 oder 2; d 0, 1, 2 oder 3 und n eine Zahl von 1 bis 6; mit der Maßgabe, daß die Gruppe -M-O- oder -M'-O- in bezug auf die Gruppe:

$$—CH{=\!=}C—\left[C(O)OR''''\right]_2$$

in meta- oder para-Stellung an den aromatischen Ring gebunden ist und die beiden Gruppen R''' die übrigen Stellungen des aromatischen Rings einnehmen.

**3.** Zusammensetzung nach Anspruch 1, wobei das fettlösliche UV-Filter vom Typ Organosiloxan mit Benzalmalonatgruppe ausgewählt ist unter:

(i) Siliconen, die der folgenden Formel (4) entsprechen:

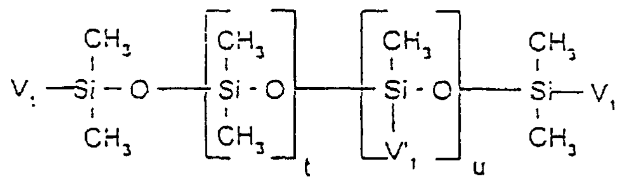 (4)

worin bedeuten:

$V'_1$ die folgende Gruppe:

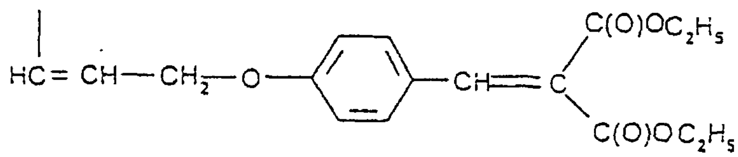

und
$V_1$ $CH_3$ oder $V'_1$;

(ii) Siliconen, die der folgenden Formel (5) entsprechen:

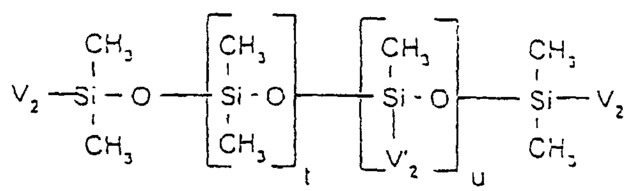 (5),

wobei $V'_2$ die folgende Gruppe bedeutet:

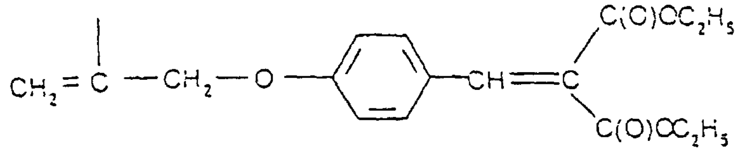

und $V_2$ $CH_3$ oder $V'_2$ bedeutet;

(iii) deren Gemischen; mit $0 \leq t \leq 100$ und $0 \leq u \leq 20$; mit der Maßgabe, daß:

- $u = 0$, wenn $V_1 = V'_1$ und/oder $V_2 = V'_2$; und
- $1 \leq u \leq 20$, wenn $V_1 = CH_3$ und/oder $V_2 = CH_3$.

**4.** Zusammensetzung nach einem der Ansprüche 1 bis 3, wobei das fettlösliche UV-Filter vom Typ Organosiloxan mit Benzalmalonatgruppe in einem Mengenanteil von 0,05 bis 10 Gew.-% und insbesondere 0,1 bis 6 Gew.-%, bezogen auf das Gesamtgewicht der Zusammensetzung, vorliegt.

**5.** Zusammensetzung nach einem der vorhergehenden Ansprüche, dadurch gekennzeichnet, daß die in Wasser unlöslichen, kationischen grenzflächenaktiven Stoffe unter den quartären Ammoniumsalzen, Fettaminen und deren Salzen ausgewählt sind.

**6.** Zusammensetzung nach einem der Ansprüche 1 bis 5, wobei die Fettamine unter Dioctylamin, Stearyldimethylamin, Palmityldimethylamin, Oleocetyldimethylamin und Amidoaminen, wie beispielsweise Stearylamidoethyldiethylamin, Behenylamidopropyldimethylamin, Stearylamidopropyldimethylamin, Oleylamidopropyldimethylamin und Stearylamidoethyldimethylamin, ausgewählt sind.

**7.** Zusammensetzung nach Anspruch 5, wobei die in Wasser unlöslichen, kationischen grenzflächenaktiven Stoffe ausgewählt sind unter:

- den quartären Ammoniumsalzen, die der folgenden allgemeinen Formel (I) entsprechen:

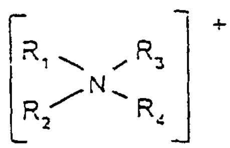

$X^-$ (I),

worin die Gruppen $R_1$ bis $R_4$, die identisch oder voneinander verschieden sein können, eine substituierte oder unsubstituierte aliphatische Gruppe mit 1 bis etwa 30 Kohlenstoffatomen, wie beispielsweise Alkyl, Alkoxy, $C_{2-6}$-Polyoxyalkylen, Alkylamid, $C_{12-22}$-Alkyl-$C_{2-6}$-amidoalkyl, $C_{12-22}$-Alkylacetat und Hydroxyalkyl, oder eine aromatische Gruppe bedeuten, wie Aryl oder Alkylaryl; und X ein Anion ist, das unter den Halogeniden, Phosphaten, Acetaten, Lactaten, Alkylsulfaten und Alkyl- oder Alkylarylsulfonaten ausgewählt ist;

- den quartären Ammoniumsalzen von Imidazolinium, beispielsweise den Verbindungen der folgenden Formel (II):

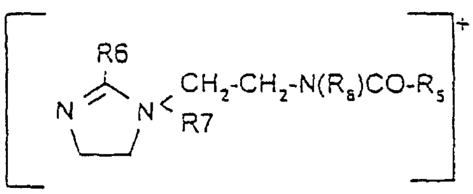

$X^-$ (II),

worin $R_5$ eine Alkenyl- oder Alkylgruppe mit 8 bis 30 Kohlenstoffatomen bedeutet, beispielsweise Derivate von Talgfettsäuren, $R_6$ ein Wasserstoffatom, eine $C_{1-4}$-Alkylgruppe oder eine Alkenylgruppe oder Alkylgruppe mit 8 bis 30 Kohlenstoffatomen bedeutet; $R_7$ eine $C_{1-4}$-Alkylgruppe ist; $R_8$ Wasserstoff oder eine $C_{1-4}$-Alkylgruppe bedeutet; und X ein Anion ist, das unter den Halogeniden, Phosphaten, Acetaten, Lactaten, Alkylsulfaten und Alkyl- oder Alkylarylsulfonaten ausgewählt ist;

- den quartären Diammoniumsalzen der Formel (III):

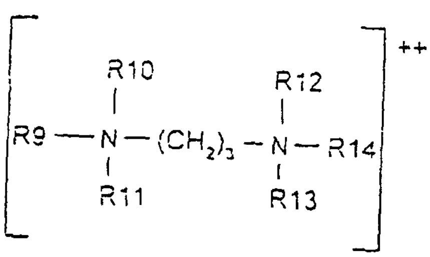

$2X^-$ (III),

worin die Gruppe $R_9$ eine aliphatische Gruppe mit etwa 16 bis 30 Kohlenstoffatomen bedeutet, die Gruppen $R_{10}$, $R_{11}$, $R_{12}$, $R_{13}$ und $R_{14}$ unter Wasserstoff oder einer Alkylgruppe mit 1 bis 4 Kohlenstoffatomen ausgewählt sind, und X ein Anion ist, das unter den Halogeniden, Acetaten, Phosphaten, Sulfaten, Nitraten und Methylsulfaten ausgewählt ist.

**8.** Zusammensetzung nach einem der Ansprüche 1 bis 7, wobei die in Wasser unlöslichen, kationischen grenzflächenaktiven Stoffe in Mengenanteilen von 0,05 bis 10 Gew.-% und insbesondere 0,1 bis 6 Gew.-%, bezogen auf das Gesamtgewicht der Zusammensetzung, verwendet werden.

**9.** Zusammensetzung nach einem der Ansprüche 1 bis 8, wobei der pH-Wert im Bereich von 3 bis 8 und vorzugsweise 4 bis 6 liegt.

**10.** Zusammensetzung nach einem der Ansprüche 1 bis 9, dadurch gekennzeichnet, daß sie mindestens eine wäßrige Phase und eine Fettphase aufweist, die entweder aus dem Organosiloxanfilter alleine oder aus einem Gemisch des Filters mit einer oder mehreren Fettsubstanzen besteht, die unter den Mineralölen, pflanzlichen Ölen, tierischen Ölen oder synthetischen Ölen, Siliconölen mit linearer oder cyclischer Struktur oder Siliconwachsen, Siliconharzen oder Silicongummis sowie deren Gemischen ausgewählt sind.

**11.** Zusammensetzung nach einem der Ansprüche 1 bis 10, dadurch gekennzeichnet, daß sie als Creme, Milch, Gel, Gel-Creme, nichtionische Vesikeldispersion, Lotion oder Spray vorliegt oder unter Druck in einer Aerosolvorrichtung konfektioniert ist.

**12.** Zusammensetzung nach einem der Ansprüche 1 bis 11, dadurch gekennzeichnet, daß sie ferner einen oder mehrere kosmetische Zusatzstoffe enthält, die unter den Färbemitteln, grenzflächenaktiven Stoffen, Polymeren, Verdickungsmitteln, Konditioniermitteln, Trübungsmitteln, Perlglanzmitteln, Wirkstoffen gegen Schuppen, Wirkstoffen gegen Haarausfall, Ceramiden, Vitaminen, Antioxidantien, Parfums, Konservierungsmitteln, reizlindernden Stoffen sowie weiteren Filtern ausgewählt sind.

**13.** Zusammensetzung nach einem der Ansprüche 1 bis 12, dadurch gekennzeichnet, daß sie als Produkt für das Haar verwendet wird.

**14.** Verfahren zur kosmetischen Behandlung zum Schutz der Haare gegen die Wirkungen der UV-Strahlung und zum Schutz ihrer natürlichen Farbe oder künstlichen Färbung, das darin besteht, auf die Haare eine wirksame Menge einer Zusammensetzung nach einem der Ansprüche 1 bis 13 aufzutragen.

**15.** Verwendung eines in Wasser unlöslichen, kationischen grenzflächenaktiven Stoffes nach einem der Ansprüche 1 bis 7 in einer Zusammensetzung für das Haar, die in Form einer wäßrigen Dispersion vorliegt und mindestens ein fettlösliches UV-Filters vom Typ Organosiloxan mit Benzalmalonatgruppe nach einem der Ansprüche 1 bis 3 enthält, um die Haare und ihre natürliche Farbe oder künstliche Färbung gegen die Wirkungen der UV-Strahlung zu schützen, oder seine Verwendung zur Herstellung dieser Zusammensetzungen für das Haar.

**16.** Verwendung eines in Wasser unlöslichen, kationischen grenzflächenaktiven Stoffes nach einem der Ansprüche 1 bis 7 in einer Zusammensetzung für das Haar, die in Form einer wäßrigen Dispersion vorliegt und mindestens ein fettlösliches UV-Filter vom Typ Organosiloxan mit Benzalmalonatgruppe nach einem der Ansprüche 1 bis 3 enthält, um das Abscheiden und/oder Fixieren des UV-Filters auf dem Haar zu verbessern, oder seine Verwendung zur Herstellung dieser Zusammensetzungen für das Haar.

## Claims

**1.** Cosmetic or dermatological composition in the form of an aqueous dispersion, characterized in that it comprises, in a cosmetically acceptable medium, at least one water-insoluble cationic surfactant and at least one liposoluble UV screening agent of the organosiloxane type containing a benzalmalonate function.

**2.** Composition according to Claim 1, in which the liposoluble agent for screening out UV radiation, of the organosiloxane type containing a benzalmalonate function, comprises at least some units corresponding to one of the two formulae (1) and (2) below:

$$O_{\frac{3-c}{2}}Si(R'')_c-M-O-C_6R'''_2H_2-CH=C-\left[C(O)OR''''\right]_2 \qquad (1)$$

$$O_{\frac{3-c}{2}}Si(R'')_c - M' - O - C_6R'''_2H_2 - CH{=}C - [C(O)OR'''']_2 \quad (2)$$

in which:

- M denotes a group of structure:

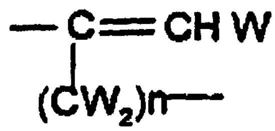

$$-CW{=}CH-(CW_2)n-$$

- M' denotes a group of structure:

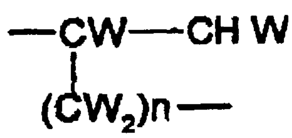

or

$$-CW_2-CHW-(CW_2)n-,$$

the other silicone units present being of structure:

$$Qd - Si - O_{\frac{4-d}{2}} \quad (3)$$

in which R'' denotes a $C_1$-$C_8$ alkyl or aryl radical; W is a hydrogen or a $C_1$-$C_5$ alkyl radical; R' ' ' is a hydrogen or a $C_1$-$C_5$ alkyl radical or a radical OW; R'''' denotes a $C_1$-$C_5$ alkyl group; Q denotes a hydrogen, a monovalent $C_1$-$C_8$ hydrocarbon-based radical or a halohydrocarbon-based group; c is equal to 0, 1 or 2; d is equal to 0, 1, 2 or 3 and n has a value from 1 to 6; with the proviso that the group -M-O- or -M'-O- is connected to the aromatic ring in a meta or para position relative to the group:

$$-CH{=}C-[C(O)OR'''']_2$$

and with the proviso that the two groups R'' occupy the other remaining positions on the aromatic ring.

3. Composition according to Claim 1, in which the liposoluble agent for screening out UV radiation, of the organosiloxane type containing a benzalmalonate function, is chosen from the group consisting of:

(i) silicones corresponding to formula (4) below:

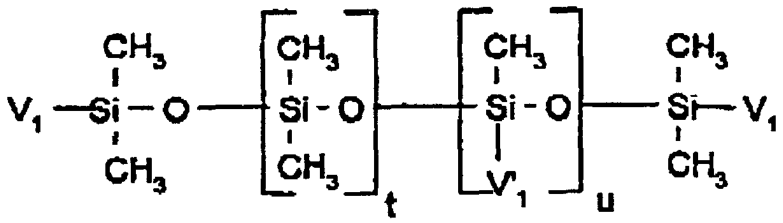

(4)

in which:

$V'_1$ denotes the group of structure:

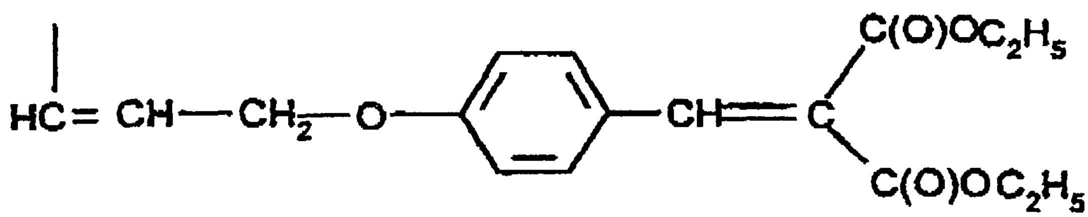

$V_1$ denotes $CH_3$ or $V'_1$;

(ii) silicones corresponding to formula (5) below:

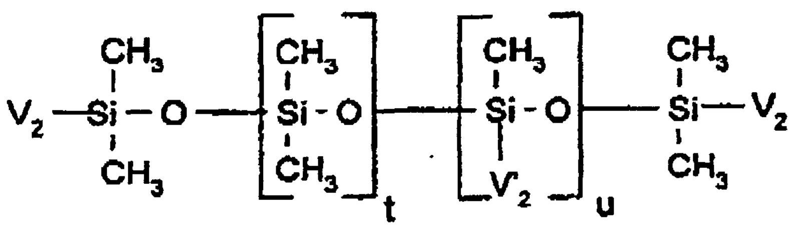

(5)

V'2 denotes the group of structure:

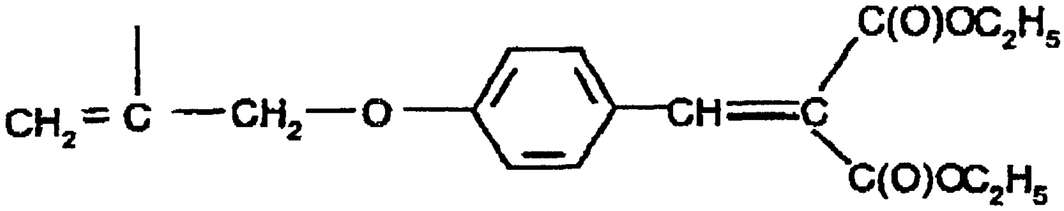

$V_2$ denotes $CH_3$ or $V'_2$;

(iii) mixtures thereof; with $0 \leq t \leq 100$ and $o \leq u \leq 20$, with the proviso that:

- when $V_1 = V'_1$ and/or $V_2 = V'_2$, then u = 0; and
- when $V_1 = CH_3$ and/or $V_2 = CH_3$, then $1 \leq u \leq 20$.

**4.** Composition according to any one of Claims 1 to 3, in which the liposoluble agent for screening out UV radiation, of the organosiloxane type containing a benzalmalonate function, is present in an amount ranging from 0.05 to 10% by weight, and more particularly from 0.1 to 6% by weight, relative to the weight of the composition.

**5.** Composition according to any one of Claims 1 to 4, in which the water-insoluble cationic surfactants are chosen

from the group consisting of quaternary ammonium salts and fatty amines and salts thereof.

**6.** Composition according to any one of Claims 1 to 5, in which the fatty amines are chosen from dioctylamine, stearyldimethylamine, palmityldimethylamine, oleocetyldimethylamine and amidoamines such as stearylamidoethyldiethylamine, behenylamidopropyldimethylamine, stearylamidopropyldimethylamine, oleylamidopropyldimethylamine and stearylamidoethyldimethylamine.

**7.** Composition according to Claim 5, in which the water-insoluble cationic surfactants are chosen from:

- quaternary ammonium salts corresponding to the general formula (I) below:

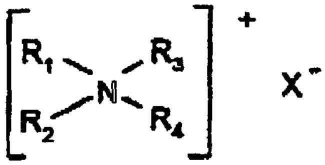

(I)

in which the radicals $R_1$ to $R_4$, which may be identical or different, represent a substituted or unsubstituted aliphatic radical comprising from 1 to about 30 carbon atoms, such as alkyl, alkoxy, polyoxy ($C_2$-$C_6$) alkylene, alkylamide, ($C_{12}$-$C_{22}$) alkylamido ($C_2$-$C_6$) alkyl, ($C_{12}$-$C_{22}$)-alkylacetate, hydroxyalkyl or an aromatic radical such as aryl or alkylaryl; X is an anion chosen from the group of halides, phosphates, acetates, lactates, alkylsulphates, alkylsulphonates or alkylarylsulphonates;

- imidazolinium quaternary ammonium salts such as, for example, that of formula (II) below:

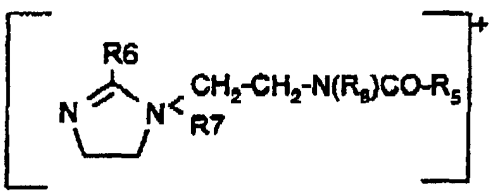

(II)

in which $R_5$ represents an alkenyl or an alkyl radical comprising from 8 to 30 carbon atoms, such as tallow fatty acid derivatives; $R_6$ represents a hydrogen atom, a $C_1$-$C_4$ alkyl radical or an alkenyl or alkyl radical comprising from 8 to 30 carbon atoms; $R_7$ represents a $C_1$-$C_4$ alkyl radical; $R_8$ represents a hydrogen atom or a $C_1$-$C_4$ alkyl radical; X is an anion chosen from the group of halides, phosphates, acetates, lactates, alkylsulphates, alkylsulphonates or alkylarylsulphonates;

- quaternary diammonium salts of formula (III):

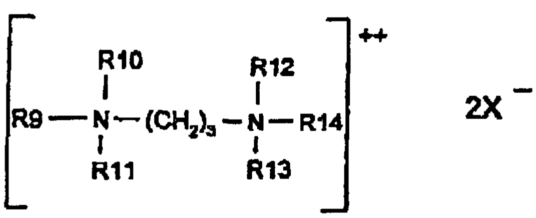

(III)

in which $R_9$ denotes an aliphatic radical comprising about 16 to 30 carbon atoms, $R_{10}$, $R_{11}$, $R_{12}$, $R_{13}$ and $R_{14}$ are chosen from hydrogen or an alkyl radical comprising from 1 to 4 carbon atoms and X is an anion chosen from the group of halides, acetates, phosphates, sulphates, nitrates and methylsulphates.

**8.** Composition according to any one of Claims 1 to 7, in which the water-insoluble cationic surfactants are used in amounts ranging from 0.05 to 10% by weight, and more particularly from 0.1 to 6% by weight, relative to the weight of the composition.

**9.** Composition according to any one of Claims 1 to 8, in which the pH ranges from 3 to 8 and more preferably from 4 to 6.

**10.** Composition according to any one of Claims 1 to 9, characterized in that it comprises at least one aqueous phase and one fatty phase consisting either solely of the screening organosiloxane, or of a mixture thereof with one or more fatty substances chosen from mineral, vegetable, animal or synthetic oils; silicone oils with a linear or cyclic structure, or silicone gums, resins or waxes, as well as mixtures thereof.

**11.** Composition according to any one of Claims 1 to 10, characterized in that it is in the form of a cream, a milk, a gel, a cream-gel, a nonionic vesicle dispersion, a lotion or a spray or it is pressurized in an aerosol device.

**12.** Composition according to any one of Claims 1 to 11, characterized in that it also contains one or more cosmetic additives chosen from dyes, surfactants, polymers, thickeners, conditioners, opacifiers, pearlescent agents, antidandruff agents, agents for preventing hair loss, ceramides, vitamins, antioxidants, fragrances, preserving agents and softeners, as well as other screening agents.

**13.** Composition according to any one of Claims 1 to 12, characterized in that it is used as a haircare product.

**14.** Cosmetic treatment process for protecting the hair against the effects of UV rays and for protecting its natural or artificially dyed colour, this process consisting in applying an effective amount of a composition as defined according to any one of Claims 1 to 13 to the hair.

**15.** Use of a water-insoluble cationic surfactant as defined in any one of Claims 1 to 7, in, or for the manufacture of, a hair composition in the form of an aqueous dispersion comprising at least one liposoluble UV screening agent of the organosiloxane type containing a benzalmalonate function as defined in any one of Claims 1 to 3, with the aim of protecting the hair and its natural or artificially dyed colour against the effects of UV radiation.

**16.** Use of a water-insoluble cationic surfactant as defined in any one of Claims 1 to 7, in, or for the manufacture of, a hair composition in the form of an aqueous dispersion comprising at least one liposoluble UV screening agent of the organosiloxane type containing a benzalmalonate function, as defined in any one of Claims 1 to 3, in order to improve the deposition and/or fixing of the said UV screening agent on the hair.